# EUROPEAN PATENT SPECIFICATION

(11) **EP 1 811 087 B1**
(45) Date of publication and mention of the grant of the patent: **16.02.2011**
(21) Application number: 05787235.0
(22) Date of filing: 02.08.2005
(51) Int. Cl.: E01F 7/00, F16F 7/14

(54) **IMPACT-ABSORBING DEVICE FOR USE IN EARTH-BANK-PROTECTION SYSTEMS**
STOSSDÄMPFUNGSVORRICHTUNG FÜR AUFSCHÜTTUNGSSCHUTZSYSTEME
DISPOSITIF AMORTISSEUR D'IMPACTS CONTENU DANS DES SYSTEMES DE PROTECTION DE TALUS

(30) Priority: 15.10.2004 ES 200402443
(43) Date of publication of application: 25.07.2007
(73) Proprietor: Malla Talud Cantabria, S.L., 39408 Barros (ES)
(72) Inventor: CASTRO FRESNO, Daniel, Escuela de Caminos, C.P., 39005 Santander (ES)
(74) Representative: Molina Garcia, Julia
(86) International application number: PCT/ES2005/000442
(87) International publication number: WO 2006/042882

(56) References cited:
- EP-A1- 0 494 046
- EP-A2- 0 208 616
- ES-U- 1 040 741
- FR-A1- 2 539 477
- FR-A1- 2 673 253
- SU-A1- 796 550
- US-A- 5 435 524
- PATENT ABSTRACTS OF JAPAN & JP 2003 239225 A (ASAHI STEEL INDUSTRY) 27 August 2003

## Description

### OBJECT OF THE INVENTION

This invention, as described in the report, is a device for absorbing impacts in slope protection systems, especially in containment screens and protection against falling stones or other bodies breaking away from hill sides to roll down the slope.

When securing land close to roads with traffic susceptible to the risk of rock landslide, defence structures are installed using cemented posts in the ground supporting a mesh net to contain any falling bodies. These structures incorporate taut anchoring cables between the hill and the fencing.

It has been verified that in the event of considerable elasticity load on this metal mesh, it is insufficient to absorb the kinetic energy, thus leading to breakage of the anchoring cables due to the sudden pull caused by large impacts, another possible effect on the mesh being considerable deformity. In order to equip these structures with a greater dynamic, anchoring cables are installed using loops or lacing with systems that allow for partial deformity of the dimensions of said loop or lacing in the event of substantial loads, thus enabling them to absorb the impact in successive stages.

The European Patent EP 0 494 046 shows us an impact absorbing device for slope protection systems with a cable forming a lacing cased in a loop or "O" shaped tube, being compressed on the outside of the ends of the tube by a tightening organ, which allows the tube to move with a resizing of the lacing when the protection system is overload.

In a further European Patent, Utility Model ES1040741V this cable applies a safety system in landslide zones, by installing cables with tubular lacing in the jackstays, made taut between the hill and the post, secured by a slack adjuster at the ends of the lacing. These cables, with identical characteristics, are also installed on the upper and lower mesh fastening.

Utility Model ES1028142V presents a anti-rock slide screen with a tightening cable with no tubular coating, with loops positioned across clip devices operating with brake elements, such that the loop becomes deformed, thus reducing its size as the safety screen is subjected to overload.

The invention presented here substantially improves on these safety systems outlined above, achieving a balanced dissipation of energy in the event of considerable impacts on the safety mesh. This is achieved with a shock absorbing device comprising two cables, each forming lacing with identical dimensions. Each cable is encased in a tube, the lacing closed off by securing the cables with a ring.

The lacings are located in opposing directions, in their upper and lower parts fitted with tightening elements for a joint fastening at these points, exerting pressure on the tubes while allowing for the tubes to shift when the cables are subject to important overloads on the mesh.

Since the system is fitted with two twin sliding cables, the ability to absorb considerable pulls is greater than in previously known systems, as it distributes the energy evenly in the lacing set up in this manner.

To complement this description and in order to provide a greater understanding of the characteristics of this invention, this descriptive report is annexed by technical drawings to illustrate the recommended modus operandi, as follows:
Figure 1: Cables incorporating the device in the recommended manner.
Figure 2: A different view of where the cables are positioned.
Figure 3: Side view of the cables with tightening elements and the retaining ring at the end of the lacing.
Figure 4: View of the cables installed in the absorbing device once the lacing is exhausted.
Figure 5: Brake cables on the device in parallel, in another set up.
Figure 6: Another view of the brake cables in parallel.
Figure 7: Side view of the cables in parallel.
Figure 8: View of the cables in parallel when the lacing has been exhausted.
Figs. 9 to 12: View of the device in an alternative set up where the tightening elements are configured to avoid any contact between the tubes, both in the interwoven mode and in parallel.

As regards the figures described, the impact absorbing device is formed from two identically sized lacing cables (1, 2) on each cable, each one being cased in a long, narrow "U" shaped tube (3, 4), its curve being formed by the curve in the lacing (5, 6).

After the cased part in the "U" shaped tube, the lacing gradually lessens in size until it joins the two cables, it being secured at this point by means of a ring (9, 10).

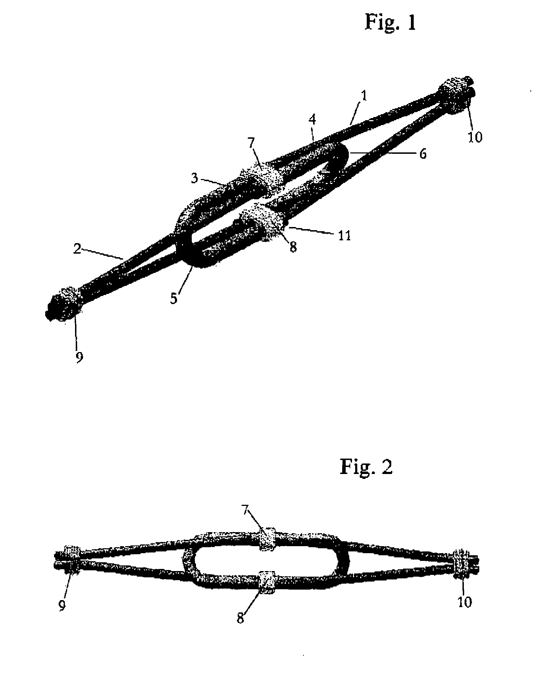

Each cable set up in this manner is located in opposite directions to each other. They are located in such a manner that they fasten each other together and in the same position as the two cased tubes (Figures 1 to 4). They are secured by tightening elements fitted on the ends of the "U" shaped tubes, applying pressure on the upper and lower part of the two lacing (7, 8). The tightening elements are to be clamps or pieces that cover the diameter of the cased tubes to distort them in their section, thus reducing their diameter at these points.

It is recommended to secure in this manner, with friction between the tubes from the two lacing (Figures 1 to 8).

As the shape of this tube is distorted due to the pressure from the tightening elements, a gap is left at the end in order to avoid the tightening element getting jammed at the edge of the cased tube (11).

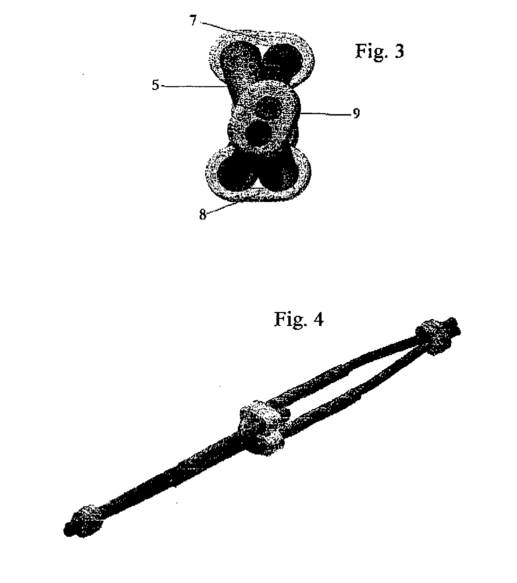

The cables are presented as recommended, mutually interlaced (Figures 1 to 4), so that the curve on each lacing is fitted on the inside perimeter of the other lacing. In this set up, when the size of the lacing is used up (Fig. 4), the tubes remain jammed at the mid point of the lacing, evenly transferring the shock forces along this point to the rest of the structure.

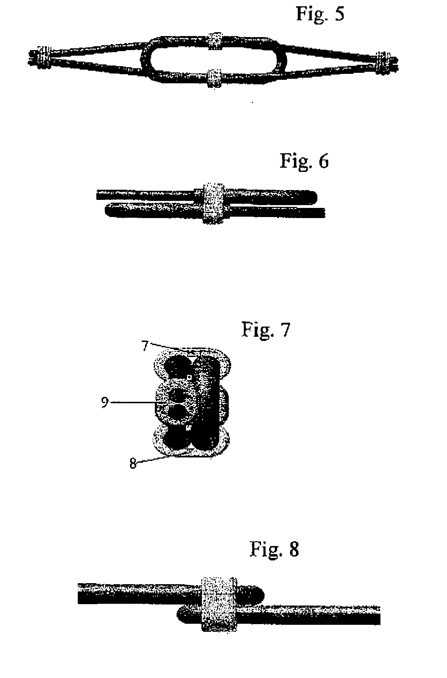

In an alternative set up, the cables are fitted in parallel and superimposed on top of each other, with no variations in the rest of the invention (Figures 5 to 8).

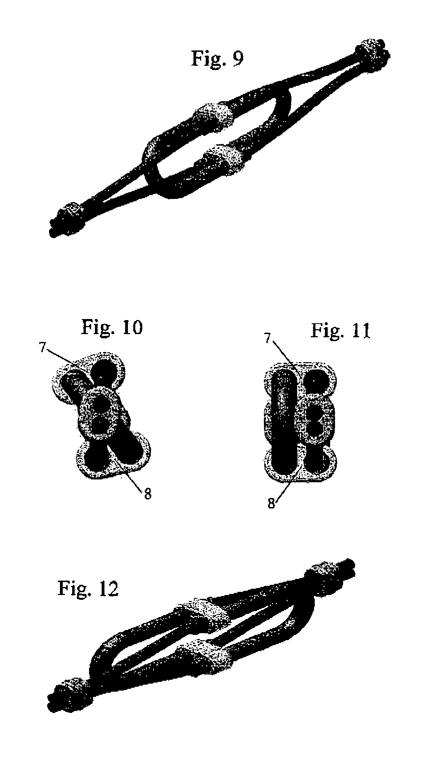

The invention may be modified with tightening elements that allow for a gap between the tubes, either by fastening them in parallel or interlacing them (Figures 9 to 12).

A further variation involves applying bare cables without inserting them into the tubes, in direct contact with the tightening elements.

The set up presented for the invention can be configured on the cables fitted on the safety screen, made taut between the hill anchoring and the protection mesh. Depending on the expected loads that a safety net will have to withstand, support cables for the mesh net may also be installed, between the posts.

In the event of shock to the protection mesh from bodies causing an overload on the cables, the shock absorbing device will allow for movement of the tubes with cased cable, forcing them to become distorted in section as they go between the tightening elements cavity, causing energy to be given off during friction. The structure of the lacing is resized until causing the total exhaustion of the same when the tightening elements reach the curve in the lacing (Fig. 4 and 8), at which point they must be replaced.

Depending on the expected loads to be withstood, the materials of the "U" shaped tubes may vary. Likewise, and for the same reasons, the diameters may also be changed. A prediction study will determine the most appropriate tightening elements for each case.

It should be understood that the invention has been described in terms of the recommended set up. Therefore, it may be subject to changes in form, size and materials, on the condition that said alterations involve no substantial variation on the characteristics of the invention as defined in the appended claims.

## Claims

1. Impact absorbing device in slope protection systems, designed to dissipate the considerable energy loads to which a protection mesh may be subjected, which may be installed taut between a hill anchoring and the protection system, or fitted on mesh support cables with posts, the impact absorbing device comprising two cables (1,2) forming independent, identical lacing that are each cased in a "U" shaped tube (3,4), the curve of which forming the curve in the lacing (5,6), positioned in opposing and interlaced directions, the lacing being fitted together in the upper and lower part by tightening elements (7,8) located at the ends of the "U" shaped tube, which tightening elements compress the tubes, distorting them in their section and in the event of overloads in the protection system, allow for the tubes to move with a resizing of the lacing.

2. Impact absorbing device, according to Claim 1, **characterized by** the lacing closing when securing the cables by means of a ring (9, 10).

3. Impact absorbing device, according to Claim 1, **characterized by** the tightening elements (7,8) that exert pressure on the tubes (3,4), there being contact between the same.

4. Impact absorbing device, according to Claim 1, **characterized by** the fact that a further variation of the same entails the tightening elements (7,8) keeping the two lacing (5,6) separate, with no friction between the tubes (3,4).

5. Impact absorbing device, according to the previous claims, **characterized by** the fact that a further variation entails arranging the lacing (5,6) in parallel and superimposed.

6. Impact absorbing device, according to the previous claims, **characterized by** the cables (1,2) being able to come into direct contact with the tightening elements (7,8) that are fitted in the mid, upper and lower part of the lacing (5,6).

## Patentansprüche

1. Aufpralldämpfungsvorrichtung in Böschungsschutzsystemen, entworfen zur Aufnahme von beträchtlichen Energielasten, denen ein Schutznetz ausgesetzt sein kann, das zwischen einer Bergverankerung und dem Schutzsystem gespannt installiert oder an den Stützkabeln mit Pfählen angebracht werden kann, wobei die Aufpralldämpfungsvorrichtung zwei Kabel (1, 2) umfasst, die unabhängige, identische Schleifen bilden, und die beide in ein U-förmiges Rohr (3, 4) gesteckt sind, wobei deren Kurve die Kurve der Schleifen (5, 6) bildet, die in entgegengesetzte Richtungen und verflochten platziert sind, wobei die Schlaufen miteinander am oberen und am unteren Teil mittels an den Enden des U-förmigen Rohrs gelegenen Klemmelemente (7, 8) gekoppelt sind, wobei diese Klemmelemente die Rohre zusammendrücken und diese dabei in Bezug auf ihren Querschnitt verformen, und wobei die Klemmelemente im Falle von Überbelastungen im Schutzsystem ermöglichen, dass sich die Rohre mit einer Neudimensionierung der Schlaufen verschieben.

2. Aufpralldämpfungsvorrichtung nach Anspruch 1, **dadurch gekennzeichnet, dass** die Schlaufe sich mit der Fixierung der Kabel durch einen Ring (9, 10) schießt.

3. Aufpralldämpfungzvorrichtung nach Anspruch 1, **dadurch gekennzeichnet, dass** die Klemmelemente (7, 8) einen Druck auf die Rohre (3, 4) ausüben, wobei ein Kontakt zwischen denselbigen besteht.

4. Aufpralldämpfungsvorrichtung nach Anspruch 1, **dadurch gekennzeichnet, dass** in einer Ausführungsvariante derselbigen die Klemmelement (7, 8) die beiden Schlaufen (5, 6) ohne Reibung zwischen den Rohren (3, 4) getrennt halten.

5. Aufpralldämpfungsvorrichtung nach den vorherigen Ansprüchen, **dadurch gekennzeichnet, dass** in einer anderen Ausführungsvariante die Schlaufen (5, 6) parallel und überlagert angeordnet sind.

6. Aufpralldämpfungsvorrichtung nach den vorherigen Ansprüchen, **dadurch gekennzeichnet, dass** die Kabel (1, 2) die im mittleren, oberen und unteren Teil der Schlaufen (5, 6) installierten Klemmelemente (7, 8) direkt kontaktieren können.

## Revendications

1. Dispositif d'absorption d'impact dans des systèmes de protection de talus, conçu pour dissiper des charges d'énergie considérables auxquelles peut être soumise une maille de protection, qui peut être installée tendue entre un ancrage de montagne et le système de protection, ou mise en place sur des câbles de support avec des poteaux, le dispositif d'absorption d'impact comprenant deux câbles (1, 2) formant des boucles indépendantes, identiques, chacun desquels est engainé dans un tube (3, 4) sous forme de "U", la courbe de ceux-ci formant la courbe des boucles (5, 6), positionnés dans des directions opposées et entrelacés, les boucles étant couplées entre elles par la partie supérieure et inférieure par le biais d'éléments de serrage (7, 8) situés aux extrémités du tube sous forme de "U", dont les éléments de serrage compressent les tubes, en les déformant dans leur section, et permettant en cas de surcharges dans le système de protection, que les tubes se déplacent avec un redimensionnement des boucles,

2. Dispositif d'absorption d'impact, selon la revendication 1, **caractérisé en ce que** la boucle se ferme au moyen d'une fixation des câbles par le biais d'une bague (9, 10).

3. Dispositif d'absorption d'impact, selon la revendication 1, **caractérisé en ce que** les éléments de serrage (7, 8) exercent de la pression sur les tubes (3, 4), en existant un contact entre ceux-ci.

4. Dispositif d'absorption d'impact, selon la revendication 1, **caractérisé en ce que**, dans une variante de mise en oeuvre de celui-ci, les éléments de serrage (7, 8) maintiennent les deux boucles (5, 6) séparées, sans friction entre les tubes (3, 4).

5. Dispositif d'absorption d'impact, selon les revendications précédentes, **caractérisé en ce que** dans une autre variante de mise en oeuvre, les boucles (5, 6) sont disposées en parallèle et superposées.

6. Dispositif d'absorption d'impact, selon les revendications précédentes, **caractérisé en ce que** les câbles (1, 2) peuvent entrer en contact direct avec les éléments de serrage (7, 8) qui sont installés dan la partie médiane, supérieure et inférieure, des boucles (5, 6).
